# FASCICULE DE BREVET EUROPEEN

(11) **EP 0 848 947 B1**
(45) Date de publication et mention de la délivrance du brevet: **11.08.1999**
(21) Numéro de dépôt: 97402781.5
(22) Date de dépôt: 19.11.1997
(51) Int. Cl.: A61K 7/42

(54) **Compositions comprenant un dérivé de dibenzoylméthane, un dérivé de 1;3.5-triazine et un benzalmalonate de diakyle et utilisations**
Mittel enthaltend ein Dibenzoylmethanederivaten, einen Triazinderivaten und einen Dialkyl-benzalmalonaten
Compositions containing a derivative of dibenzoylmethane, a derivative of 1,3,4-triazine and an dialkly benyalmalonate

(30) Priorité: 17.12.1996 FR 9615511
(43) Date de publication de la demande: 24.06.1998
(73) Titulaire: L'OREAL, 75008 Paris (FR)
(72) Inventeur: Allard, Delphine, 92700 Colombes (FR); Forestier, Serge, 77410 Claye Souilly (FR)
(74) Mandataire: Miszputen, Laurent

(56) Documents cités:
- EP-A- 0 100 651
- EP-A- 0 350 386
- EP-A- 0 507 691
- EP-A- 0 689 828

## Description

La présente invention concerne de nouvelles compositions cosmétiques et/ou dermatologiques (ci-après appelées compositions antisolaires) destinées à la protection de la peau et/ou des cheveux contre les rayonnements UV, en particulier le rayonnement solaire. Plus précisément, elle concerne de nouvelles compositions cosmétiques et/ou dermatologiques présentant une photostabilité améliorée et comprenant, dans un support cosmétiquement et/ou dermatologiquement acceptable, l'association de trois filtres particuliers.

L'invention conceme également l'utilisation de ces compositions dans les domaines cosmétique et/ou dermatologique.

On sait que les radiations lumineuses de longueurs d'onde comprises entre 280 nm et 400 nm permettent le brunissement de l'épiderme humain, et que les rayons de longueurs d'onde plus particulièrement comprises entre 280 et 320 nm, connus sous la dénomination d'UV-B, provoquent des érythèmes et des brûlures cutanées qui peuvent nuire au développement du bronzage naturel. Pour ces raisons ainsi que pour des raisons esthétiques, il existe une demande constante de moyens de contrôle de ce bronzage naturel en vue de contrôler ainsi la couleur de la peau ; il convient donc de filtrer ce rayonnement UV-B.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photo-allergiques. Ainsi, pour des raisons esthétiques et cosmétiques telles que la conservation de l'élasticité naturelle de la peau par exemple, de plus en plus de gens désirent contrôler l'effet des rayons UV-A sur leur peau. Il est donc souhaitable de filtrer aussi le rayonnement UV-A.

Ainsi, dans le but d'assurer une protection de la peau et des cheveux contre l'ensemble du rayonnement UV qui soit la plus complète et la plus efficace possible, on utilise généralement dans la fabrication des compositions antisolaires des associations de filtres actifs dans l'UVA et de filtres actifs dans l'UVB.

A cet égard, une famille de filtres UV-A particulièrement intéressante est actuellement constituée par les dérivés du dibenzoylméthane, et notamment le 4-tert.-butyl-4'-méthoxy dibenzoylméthane, qui présentent en effet un fort pouvoir d'absorption intrinsèque. Ces dérivés du dibenzoylméthane, qui sont maintenant des produits bien connus en soi à titre de filtres actifs dans l'UV-A, sont notamment décrits dans les demandes de brevets français FR-A-2326405 et FR-A-2440933, ainsi que dans la demande de brevet européen EP-A-114607 ; le 4-(ter-butyl) 4'-méthoxy dibenzoylméthane est par ailleurs actuellement proposé à la vente sous la dénomination commerciale de "PARSOL 1789" par la Société GIVAUDAN.

De même, les dérivés de 1,3,5-triazine, et en particulier la 2,4,6-tris[p-(2'-éthylnexyl-1'-oxycarbonyl)anilino]-1,3,5-triazine, vendue sous la dénomination commerciale 〈〈 UVINUL T150 〉〉 par la société BASF, possèdent un fort pouvoir absorbant des UVB et il serait donc très intéressant de pouvoir les utiliser en association avec le 4-tert-butyl-4 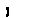 -méthoxydibenzoylméthane cité ci-dessus dans le but d'obtenir des produits offrant une protection large et efficace dans l'ensemble du rayonnement UV (voir EP-A-0 689 828).

Toutefois, la Demanderesse a constaté que ces dérivés de 1,3,5-triazine, lorsqu'ils sont en présence de dérivés du dibenzoylméthane, en particulier du 4-tert-butyl-4 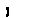 -méthoxydibenzoylméthane, et sous irradiation UV, présentent l'inconvénient de se dégrader chimiquement de façon importante. Dans ces conditions, l'association des deux filtres ne permet plus une protection solaire large prolongée de la peau et des cheveux.

Or, à la suite d'importantes recherches menées dans le domaine de la photoprotection évoqué ci-dessus, la Demanderesse a maintenant découvert que l'introduction d'un benzalmalonate de dialkyle dans une composition contenant un dérivé de dibenzoylméthane, en particulier du 4-tert-butyl-4 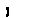 -méthoxydibenzoyl-méthane, en association avec au moins un dérivé de 1,3,5-triazine, et en particulier avec la 2,4,6-tris[p-(2'-éthylhexyl-1'-oxycarbonyl)anilino]-1,3,5-triazine, permettait d'améliorer de façon tout à fait remarquable la photostabilité de ce dérivé de 1,3,5-triazine au sein de telles compositions, et donc l'efficacité globale de ces compositions.

La présente invention a donc pour objet de nouvelles compositions cosmétiques et/ou dermatologiques comprenant, dans un support cosmétiquement et/ou dermatologiquement acceptable, i) un dérivé de dibenzoylméthane, ii) au moins un dérivé de 1,3,5-triazine répondant à la formule (I) suivante : 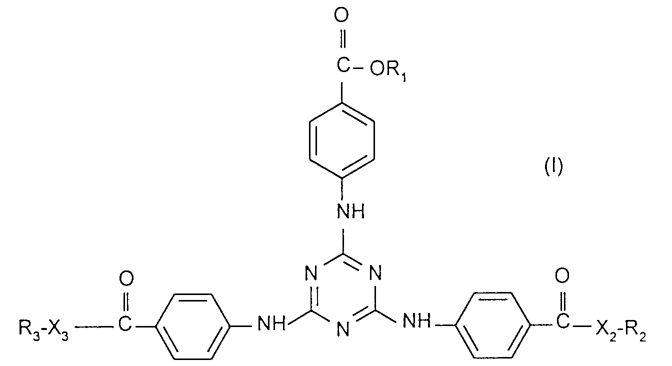 dans laquelle :
- X₂ et X₃, identiques ou différents, représentent l'oxygène ou le radical -NH-;
- R₁, R₂ et R₃, identiques ou différents, sont choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈ ; un radical cycloalkyle en C₅-C₁₂ éventuellement substitué par un ou plusieurs radicaux alkyles en C₁-C₄ ; un radical polyoxyéthyléné comprenant de 1 à 6 unités d'oxyde d'éthylène et dont le groupe OH terminal est méthylé; un radical de formule (II), (III) ou (IV) suivantes : 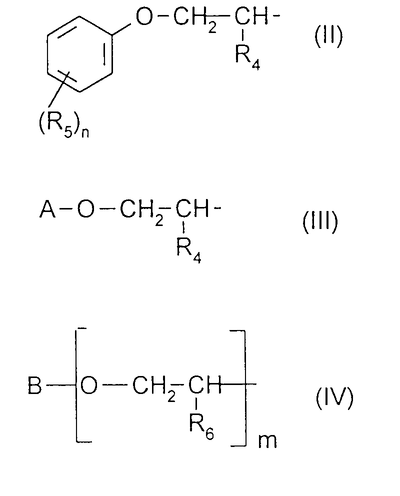 dans lesquelles :
- R₄ est l'hydrogène ou un radical méthyle ;
- R₅ est un radical alkyle en C₁-C₉ ;
- n est un nombre entier allant de 0 à 3 ;
- m est un nombre entier allant de 1 à 10 ;
- A est un radical alkyle en C₄-C₈ ou un radical cycloalkyle en C₅-C₈ ;
- B est choisi parmi : un radical alkyle linéaire ou ramifié en C₁-C₈ ; un radical cycloalkyle en C₅-C₈ ; un radical aryle éventuellement substitué par un ou plusieurs radicaux alkyles en C₁-C₄ ;
- R₆ est l'hydrogène ou un radical méthyle,
   et iii) au moins un benzalmalonate de dialkyle de formule (V) suivante :

   C₆(R₇)₃H₂-CH=C-[C(O)OR₈]₂ (V)

   dans laquelle :
- R₇ représente indépendamment un atome d'hydrogène, un radical hydroxyle, un radical alkyle en C₁-C₈, un radical alcényle en C₂-C₈, un radical alcynyle en C₂-C₈, un radical alcoxy en C₁-C₈, un radical alcényloxy en C₂-C₈, un radical alcynyloxy en C₂-C₈ ;
- R₈ représente un radical alkyle en C₁-C₈.

Ainsi, selon la présente invention, on peut réaliser des compositions cosmétiques et/ou dermatologiques contenant un dérivé de dibenzoylméthane, en particulier du 4-tert-butyl-4 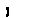 -méthoxydibenzoylméthane, en association avec au moins un dérivé de 1,3,5-triazine, compositions dans lesquelles la concentration en dérivé de 1,3,5-triazine reste relativement constante même si ces compositions sont soumises à l'action de la lumière.

Par ailleurs, les benzalmalonates de dialkyle utilisés dans le cadre de la présente invention présentent l'avantage de posséder un bon pouvoir filtrant intrinsèque qui contribue à la protection contre les UV conférée par les compositions, et, de plus, l'ensemble du système filtrant [dérivé de dibenzoylméthane + dérivé de 1,3,5-triazine + benzalmalonate de dialkyle] s'avère présenter globalement une très bonne stabilité sous l'action des UV (photostabilité), ce qui constitue un autre avantage supplémentaire des compositions selon l'invention.

La présente invention a encore pour objet l'utilisation d'un benzalmalonate de dialkyle tel que défini ci-dessus dans, ou pour la fabrication de, compositions cosmétiques et/ou dermatologiques contenant un dérivé de dibenzoylméthane, en particulier du 4-tert-butyl-4 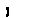 -méthoxydibenzoylméthane, en association avec au moins un dérivé de 1,3,5-triazine tel que défini ci-dessus en vue d'améliorer dans lesdites compositions la stabilité au rayonnement UV (photostabilité) dudit dérivé de 1,3,5-triazine.

La présente invention a également pour objet un procédé pour améliorer la stabilité au rayonnement UV (photostabilité), et donc l'efficacité, d'une composition cosmétique et/ou dermatologique comprenant un dérivé de dibenzoylméthane, en particulier du 4-tert-butyl-4 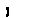 -méthoxydibenzoylméthane, et un dérivé de 1,3,5-triazine tel que défini ci-dessus, en particulier la 2,4,6-tris[p-(2'-éthylhexyl-1'-oxycarbonyl)anilino]-1,3,5-triazine, ledit procédé consistant à introduire dans ladite composition une quantité efficace d'un benzalmalonate de dialkyle tel que défini ci-dessus.

Par quantité efficace de benzalmalonate de dialkyle, on entend une quantité suffisante pour obtenir une amélioration notable et significative de la photostabilité du ou des dérivés de 1,3,5-triazine contenus dans la composition. Cette quantité minimale en agent stabilisant à mettre en oeuvre, qui peut varier selon la nature du support cosmétiquement acceptable retenu pour la composition, peut être déterminée sans aucune difficulté au moyen d'un test classique de mesure de photostabilité, tel que celui donné dans les exemples ci-après.

D'autres caractéristiques, aspects et avantages de la présente invention apparaîtront à la lecture de la description détaillée qui va suivre.

Comme indiqué précédemment, les dérivés du dibenzoylméthane utilisables selon la présente invention sont des produits déjà bien connus en soi et décrits notamment dans les documents FR-A-2326405, FR-A-2440933 et EP-A-114607 précités.

Selon la présente invention, on peut bien entendu mettre en oeuvre un ou plusieurs dérivés du dibenzoylméthane.

Parmi les dérivés du dibenzoylméthane utilisables selon la présente invention, on peut notamment citer, de manière non limitative :
- le 2-méthyldibenzoylméthane,
- le 4-méthyldibenzoylméthane,
- le 4-isopropyldibenzoylméthane,
- le 4-tert.-butyldibenzoylméthane,
- le 2,4-diméthyldibenzoylméthane,
- le 2,5-diméthyldibenzoylméthane,
- le 4,4'-diisopropyldibenzoylméthane,
- le 4-tert.-butyl-4'-méthoxydibenzoylméthane,
- le 2-méthyl-5-isopropyl-4'-méthoxydibenzoylméthane,
- le 2-méthyl-5-tert.-butyl-4'-méthoxydibenzoylméthane,
- le 2,4-diméthyl-4'-méthoxydibenzoylméthane,
- le 2,6-diméthyl-4-tert.-butyl-4'-méthoxydibenzoylméthane,
- le 4,4 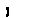 -diméthoxydibenzoylméthane.

Parmi les dérivés du dibenzoylméthane mentionnés ci-dessus, on préfère tout particulièrement, selon la présente invention, mettre en oeuvre le 4-tert.-butyl-4'-méthoxy dibenzoylméthane, notamment celui proposé à la vente sous la dénomination commerciale de "PARSOL 1789" par la Société GIVAUDAN, ce filtre répondant donc à la formule développée suivante :

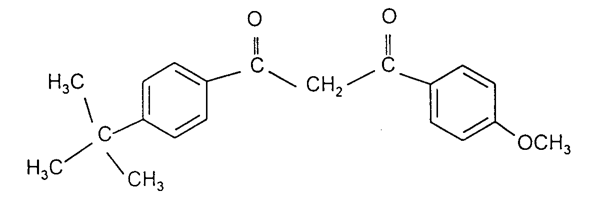

Un autre dérivé du dibenzoylméthane préféré selon la présente invention est le 4-isopropyl-dibenzoylméthane, filtre vendu sous la dénomination de "EUSOLEX 8020" par la Société MERCK, et répondant à la formule développée suivante :

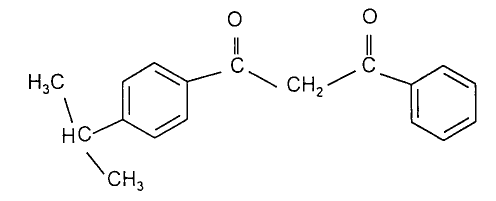

Les dérivés de dibenzoylméthane peuvent être présents dans les compositions de l'invention à une teneur allant de 0,2 % à 15 % en poids, par rapport au poids total de la composition. De préférence, cette teneur va de 0,2 % à 10 %.

Un deuxième composé des compositions visées par la présente invention est un dérivé particulier de 1,3,5-triazine. Ainsi, les dérivés de 1,3,5-triazine utilisables dans le cadre de la présente invention sont choisis parmi ceux répondant à la formule (I) suivante : 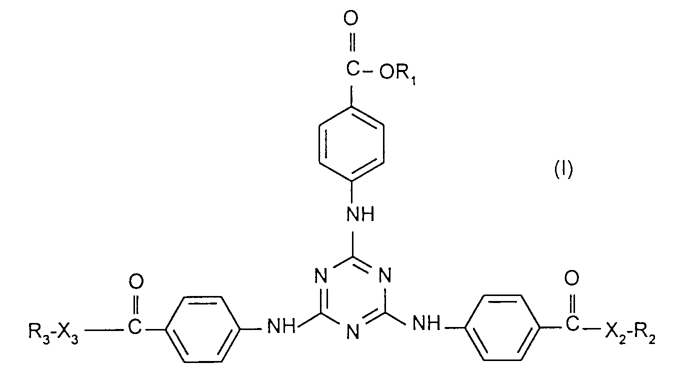 dans laquelle :
- X₂ et X₃, identiques ou différents, représentent l'oxygène ou le radical -NH-;
- R₁, R₂ et R₃, identiques ou différents, sont choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄; un radical polyoxyéthyléné comprenant de 1 à 6 unités d'oxyde d'éthylène et dont le groupe OH terminal est méthylé; un radical de formule (II), (III) ou (IV) suivantes : 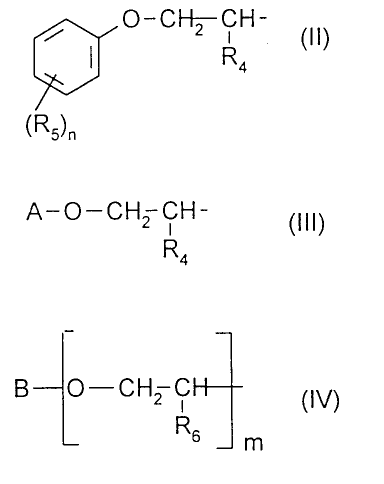 dans lesquelles :
- R₄ est l'hydrogène ou un radical méthyle ;
- R₅ est un radical alkyle en C₁-C₉ ;
- n est un nombre entier allant de 0 à 3 ;
- m est un nombre entier allant de 1 à 10 ;
- A est un radical alkyle en C₄-C₈ ou un radical cycloalkyle en C₅-C₈ ;
- B est choisi parmi : un radical alkyle linéaire ou ramifié en C₁-C₈ ; un radical cycloalkyle en C₅-C₈ ; un radical aryle éventuellement substitué par un ou plusieurs radicaux alkyles en C₁-C₄ ;
- R₆ est l'hydrogène ou un radical méthyle.

Bien entendu, dans la définition ci-dessus, lorsque X₂ et/ou X₃ représentent un radical -NH-, alors le ou les radicaux R₂ et/ou R₃ correspondants sont différents d'un métal alcalin ou d'un radical ammonium.

Une première famille plus particulièrement préférée de dérivés de 1,3,5-triazine est celle, notamment décrite dans le document EP-A-517104, des 1,3,5-triazines répondant à la formule (I) ci-dessus et présentant l'ensemble des caractéristiques suivantes :
- X₂ et X₃ sont identiques et représentent l'oxygène ;
- R₁ est choisi parmi : un radical cycloalkyle en C₅-C₁₂ éventuellement substitué par un ou plusieurs radicaux alkyles en C₁-C₄ ; un radical de formule (II), (III) ou (IV) ci-dessus dans lesquelles :
   - B est un radical alkyle en C₁-C₄ ;
   - R₆ est le radical méthyle;
- R₂ et R₃, identiques ou différents, sont choisis parmi : l'hydrogène ; un métal alcalin ; un radical ammonium éventuellement substitué par un ou plusieurs radicaux alkyles ou hydroxyalkyles ; un radical alkyle linéaire ou ramifié en C₁-C₁₈ ; un radical cycloalkyle en C₅-C₁₂ éventuellement substitué par un ou plusieurs radicaux alkyles en C₁-C₄ ; un radical de formule (II), (III) ou (IV) ci-dessus dans lesquelles :
   - B est un radical alkyle en C₁-C₄ ;
   - R₆ est le radical méthyle.

Une deuxième famille préférée de dérivés de 1,3,5-triazine selon l'invention est celle, notamment décrite dans le document EP-A-570838, des 1,3,5-triazines répondant à la formule (I) et présentant l'ensemble des caractéristiques suivantes :
- X₃ est le radical -NH-;
- R₃ est choisi parmi : un radical alkyle linéaire ou ramifié en C₁-C₁₈ ; un radical cycloalkyle en C₅-C₁₂ éventuellement substitué avec un ou plusieurs radicaux alkyles en C₁-C₄ ;
- R₁ est choisi parmi : l'hydrogène ; un métal alcalin ; un radical ammonium ; un radical de formule (IV) ; un radical alkyle linéaire ou ramifié en C₁-C₁₈ ; un radical cycloalkyle en C₅-C₁₂ éventuellement substitué avec un ou plusieurs radicaux alkyles en C₁-C₄ ;
- si X₂ est le radical -NH-, alors R₂ est choisi parmi : un radical alkyle linéaire ou ramifié en C₁-C₁₈ ; un radical cycloalkyle en C₅-C₁₂ éventuellement substitué avec un ou plusieurs radicaux alkyles en C₁-C₄ ;
- si X₂ est l'oxygène, alors R₂ est choisi parmi l'hydrogène ; un métal alcalin ; un radical ammonium ; un radical de formule (IV) ; un radical alkyle linéaire ou ramifié en C₁-C₁₈ ; un radical cycloalkyle en C₅-C₁₂ éventuellement substitué avec un ou plusieurs radicaux alkyles en C₁-C₄.

Une 1,3,5-triazine particulièrement préférée de cette deuxième famille est celle répondant à la formule suivante : 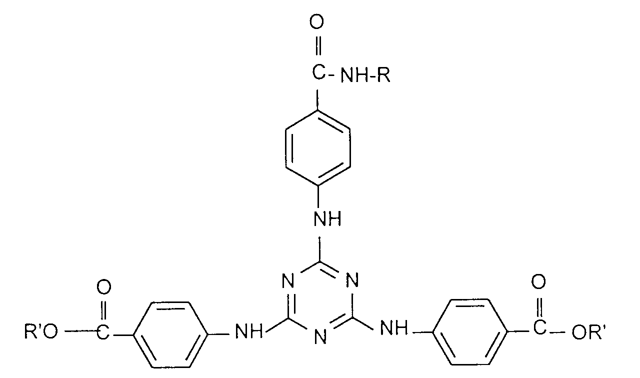 dans laquelle R 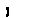 désigne un radical éthyl-2 hexyle et R désigne un radical tert-butyle.

Une troisième famille préférée de composés est celle, notamment décrite dans le document US4,724,137, des 1,3,5-triazines répondant à la formule (I) et présentant l'ensemble des caractéristiques suivantes :
- X₂ et X₃ sont identiques et représentent l'oxygène ;
- R₁, R₂ et R₃ sont identiques et représentent un radical alkyle en C₆-C₁₂ ou un radical polyoxyéthyléné comprenant de 1 à 6 unités d'oxyde d'éthylène et dont le groupe OH terminal est méthylé.

Une 1,3,5-triazine particulièrement préférée de cette troisième famille est la 2,4,6-tris[p-(2'-éthylhexyl-1'-oxycarbonyl)anilino]-1,3,5-triazine qui est un filtre connu en soi, actif dans l'UV-B, se présentant sous une forme solide, et qui est vendu notamment sous la dénomination commerciale de "UVINUL T 150" par la Société BASF. Ce produit répond à la formule suivante : 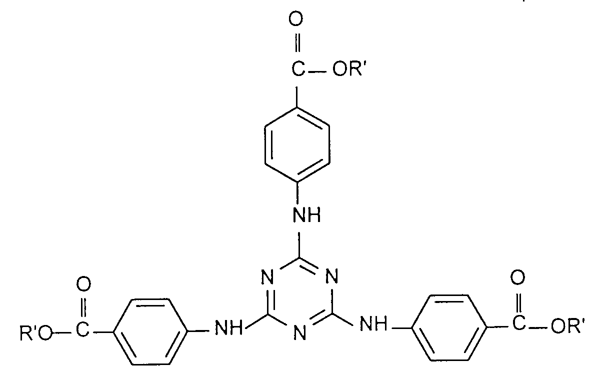 dans laquelle R 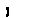 désigne un radical 2-éthyl hexyle.

Le ou les dérivés de 1,3,5-triazine sont généralement présents dans les compositions de l'invention à une teneur pouvant aller de 0,5 % à 20 %, de préférence de 1 % à 10 % en poids, par rapport au poids total de la composition.

Un troisième composé absolument essentiel des compositions selon l'invention est un composé de la famille des benzalmalonates de dialkyle. Plus précisément, les benzalmalonates de dialkyle utilisables dans la présente invention répondent à la formule (V) suivante :

C₆(R₇)₃H₂-CH=C-[C(O)OR₈]₂ (V)

dans laquelle :
- R₇ représente indépendamment un atome d'hydrogène, un radical hydroxyle, un radical alkyle en C₁-C₈, un radical alcényle en C₂-C₈, un radical alcynyle en C₂-C₈, un radical alcoxy en C₁-C₈, un radical alcényloxy en C₂-C₈, un radical alcynyloxy en C₂-C₈,
- R₈ représente un radical alkyle en C₁-C₈.

Dans une forme préférée de réalisation de l'invention, les benzalmalonates de dialkyle répondent à la formule (VI) ci-dessous : 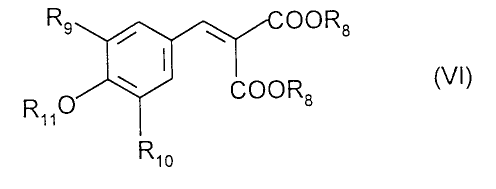 dans laquelle :
- R₈ a la même signification qu'à la formule (V) ci-dessus,
- R₉ et R₁₀, identiques ou différents, représentent indépendamment un atome d'hydrogène ou un radical alcoxy en C₁-C₈,
- R₁₁ représente un radical alkyle en C₁-C₈.

Parmi les benzalmalonates de dialkyle de formule (VI), on préfère plus particulièrement les composés suivants :
- 4 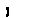 -méthoxy-benzalmalonate de diéthyle,
- 4 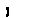 -tert.-butoxy-benzalmalonate de diéthyle,
- 4 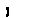 -méthoxy-benzalmalonate de diisopropyle,
- 4 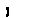 -méthoxy-benzalmalonate de di-2-éthylhexyle,
- 4 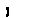 -n-butoxy-3 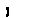 -méthoxy-benzalmalonate de diéthyle,
- 4 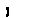 -n-butoxy-3 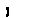 -méthoxy-benzalmalonate de diisopropyle,
- 3 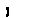 ,4 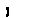 -diméthoxy-benzalmalonate de di-2-éthylhexyle,
- 3 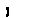 ,4 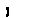 ,5 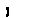 -triméthoxy-benzalmalonate de diisopropyle,
- 4 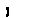 -méthoxy-benzalmalonate de diisoamyle,
- 4 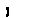 -n-hexyloxy-benzalmalonate de diéthyle.

Ces benzalmalonates de dialkyle ainsi que leurs préparations sont décrits dans les demandes EP-A-538431 et EP-A-392882.

Ainsi, lorsqu'on ajoute en quantité suffisante un benzalmalonate de dialkyle tel que défini ci-dessus à une composition antisolaire contenant un dérivé de dibenzoylméthane, en particulier du 4-tert-butyl-4 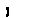 -méthoxydibenzoylméthane, et un dérivé de 1,3,5-triazine tel que défini ci-dessus, on observe une augmentation de la stabilité dudit dérivé de 1,3,5-triazine à la lumière, et donc une amélioration de l'efficacité de la composition antisolaire au cours du temps.

De préférence, le benzalmalonate de dialkyle est présent dans les compositions selon l'invention à une teneur au moins égale à 0,5 % en poids, par rapport au poids total de la composition. De préférence encore, cette teneur va de 0,5 % à 20 % en poids, par rapport au poids total de la composition.

Les compositions cosmétiques et/ou dermatologiques visées par la présente invention peuvent bien entendu contenir un ou plusieurs filtres solaires complémentaires actifs dans l'UVA et/ou l'UVB (absorbeurs), hydrophiles ou lipophiles, autres bien sûr que les trois filtres mentionnés ci-avant. Ces filtres complémentaires peuvent être notamment choisis parmi les dérivés cinnamiques, les dérivés salicyliques, les dérivés du benzylidène camphre, les dérivés de benzimidazole, les dérivés de triazine autres que ceux ci-avant mentionnés, les dérivés de la benzophénone, les dérivés de β,β 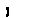 -diphénylacrylate, les dérivés de l'acide p-aminobenzoïque, les polymères filtres et silicones filtres décrits dans la demande WO-93/04665. D'autres exemples de filtres organiques sont donnés dans la demande de brevet EP-A 0 487 404.

Les compositions selon l'invention peuvent également contenir des agents de bronzage et/ou de brunissage artificiels de la peau (agents autobronzants), tels que par exemple de la dihydroxyacétone (DHA).

Les compositions cosmétiques et/ou dermatologiques selon l'invention peuvent encore contenir des pigments ou bien encore des nanopigments (taille moyenne des particules primaires : généralement entre 5 nm et 100 nm, de préférence entre 10 et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs bien connus en soi agissant par blocage physique (réflexion et/ou diffusion) du rayonnement UV. Des agents d'enrobage classiques sont par ailleurs l'alumine et/ou le stéarate d'aluminium. De tels nanopigments d'oxydes métalliques, enrobés ou non enrobés, sont en particulier décrits dans les demande de brevets EP-A- 518772 et EP-A-518773.

Les compositions conformes à la présente invention peuvent comprendre en outre des adjuvants cosmétiques classiques notamment choisis parmi les corps gras, les solvants organiques, les épaississants ioniques ou non ioniques, les adoucissants, les antioxydants, les agents anti radicaux libres, les opacifiants, les stabilisants, les émollients, les silicones , les α-hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants, ou tout autre ingrédient habituellement utilisé dans le domaine cosmétique et/ou dermatologique, en particulier pour la fabrication de compositions antisolaires sous forme d'émulsions.

Les corps gras peuvent être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (vaseline); végétales (huile d'amande douce, de macadamia, de pépin de cassis, de jojoba) ; synthétiques comme le perhydrosqualène, les alcools, les acides ou les esters gras (comme le benzoate d'alcools en C₁₂-C₁₅ vendu sous la dénomination commerciale 〈〈 Finsolv TN 〉〉 par la société Finetex, le palmitate d'octyle, le lanolate d'isopropyle, les triglycérides dont ceux des acides caprique/caprylique), les esters et éthers gras oxyéthylénés ou oxypropylénés; siliconées (cyclométhicone, polydiméthyl-siloxanes ou PDMS) ou fluorées, les polyalkylènes.

Comme composés cireux, on peut citer la paraffine, la cire de carnauba, la cire d'abeille, l'huile de ricin hydrogénée.

Parmi les solvants organiques, on peut citer les alcools et polyols inférieurs.

Les épaississants peuvent être choisis notamment parmi les acides polyacryliques réticulés, les gommes de guar et celluloses modifiées ou non telles que la gomme de guar hydroxypropylée, la méthylhydroxyéthylcellulose et l'hydroxypropylméthyl cellulose.

Bien entendu, l'homme de l'art veillera à choisir le ou les éventuels composés complémentaires cités ci-dessus (en particulier les filtres complémentaires) et/ou leurs quantités de manière telle que les propriétés avantageuses attachées intrinsèquement à l'association ternaire conforme à l'invention ne soient pas, ou substantiellement pas, altérées par la ou les adjonctions envisagées.

Les compositions selon l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, ou sous la forme d'un gel ou d'un gel crème, de poudre, de bâtonnet solide et éventuellement être conditionnée en aérosol et se présenter sous forme de mousse ou de spray.

De préférence, les compositions selon l'invention se présentent sous la forme d'une émulsion huile-dans-eau.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2315991 et FR 2416008).

La composition cosmétique et/ou dermatologique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de stick, de mousse aérosol ou de spray.

Lorsque la composition cosmétique selon l'invention est utilisée pour la protection des cheveux, elle peut se présenter sous forme de shampooing, de lotion, de gel, d'émulsion, de dispersion vésiculaire non ionique, de laque pour cheveux et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95 % en poids, de préférence de 70 à 90 % en poids, par rapport à l'ensemble de la formulation, la phase huileuse (comprenant notamment les filtres lipophiles) de 5 à 50 % en poids, de préférence de 10 à 30 % en poids, par rapport à l'ensemble de la formulation, et le ou les (co)émulsionnant(s) de 0,5 à 20 % en poids, de préférence de 2 à 10% en poids, par rapport à l'ensemble de la formulation.

Un exemple concret, mais nullement limitatif, illustrant l'invention, va maintenant être donné.

### EXEMPLE :

On a réalisé quatre émulsions huile-dans-eau A, B, C et D dont le support commun présente la composition suivante (les quantités sont exprimées en % de poids par rapport au poids total de la composition) :

| | |
|---|---|
| - mélange d'alcool cétylstéarylique et d'alcool cétylstéarylique oxyéthyléné (33 OE) 80/20 vendu sous la dénomination commerciale 〈〈 DEHSCONET 390 〉〉 par Tensia | 7 % |
| - mélange de mono et distéarate de glycérol vendu sous la dénomination commerciale 〈〈 CERASYNTH SD 〉〉 par ISP | 2 % |
| - alcool cétylique | 1,5 % |
| - polydiméthylsiloxane vendu sous la dénomination commerciale 〈〈 DC 200 Fluid 〉〉 par Dow Corning | 1,5 % |
| - benzoate d'alcools C12/C15 vendu sous la dénomination commerciale 〈〈 FINSOLV TN 〉〉 par Finetex | 15 % |
| - acide éthylène diamine tétracétique, sel disodique, 2 H₂0 | 0,1 % |
| - glycérine | 20 % |
| - conservateurs qs | |
| - eau déminéralisée qsp | 100 % |

L'émulsion A (comparative) comprend en outre un dérivé de 1,3,5 triazine qui est la 2,4,6-tris[p-(2'-éthylhexyl-1'-oxycarbonyl)anilino]-1,3,5-triazine (UVINUL T150). L'émulsion B, également comparative, contient de l'UVINUL T 50 en association avec du 4-tert-butyl-4 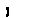 -méthoxydibenzoylméthane (PARSOL 1789). L'émulsion C, selon l'invention, comprend, outre de l'UVINUL T150 et du PARSOL 1789, du 4 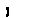 -méthoxybenzalmalonate de diisoamyle (appelé ci-après composé (c₁)). L'émulsion D, comparative, comprend quant à elle de l'UVINUL T150 en association avec le PARSOL 1789 mais avec un filtre UVB classique qui est l'octylméthoxycinnamate vendu sous la dénomination commerciale 〈〈 PARSOL MCX 〉〉 par la société GIVAUDAN.

Les compositions des émulsions A, B, C et D au niveau des différents filtres cités ci-dessus qu'elles contiennent sont rassemblées dans le tableau (I) ci-dessous (les quantités sont exprimées en % de poids par rapport au poids total de la composition) :

**Tableau (I)**

| Filtre | Emulsion A (comparative) | Emulsion B (comparative) | Emulsion C (invention) | Emulsion D (comparative) |
|---|---|---|---|---|
| UVINUL T 150 | 1,5 % | 1,5 % | 1,5 % | 1,5 % |
| PARSOL 1789 | - | 0,5 % | 0,5 % | 0,5 % |
| composé c₁ | - | - | 10 % | - |
| PARSOL MCX | - | - | - | 10 % |

Pour chacune de ces émulsions, on a déterminé le pourcentage de 2,4,6-tris[p-(2'-éthylhexyl-1'-oxycarbonyl)anilino]-1,3,5-triazine résiduelle après irradiation par des UV selon le protocole suivant : pour chaque formule, on a préparé quatre échantillons témoins et quatre échantillons tests. On a déposé sur des plaques de PMMA (polyméthacrylate de méthyle) dépolies, préalablement rincées à l'eau puis séchées, 16 mg de formule qu'on a étalée sur une surface de 2 cm x 4 cm. Puis on a irradié les plaques (SUNTEST CPS Heraeus) pendant 4 heures dans une enceinte dont la température est régulée aux environs de 35-40 °C afin de simuler une irradiation UV naturelle en conservant les plaques témoins à l'obscurité pendant le temps d'irradiation des autres plaques.

On a ensuite dosé les échantillons de la manière suivante : on a procédé à l'extraction des filtres en immergeant chaque plaque dans 55 ml d'éthanol afin de solubiliser les filtres. Les plaques et le solvant contenant les filtres ont ensuite été traités aux ultrasons pendant 5 minutes pour assurer une extraction efficace. Les solutions obtenues sont analysées par chromatographie en phase liquide haute performance.

Pour chaque formule testée, le taux de 2,4,6-tris[p-(2'-éthylhexyl-1'-oxycarbonyl)anilino]-1,3,5-triazine résiduelle après irradiation est donné par le rapport de sa concentration dans l'échantillon irradié à sa concentration dans l'échantillon non irradié.

Les résultats en pourcentage de 2,4,6-tris[p-(2'-éthylhexyl-1'-oxycarbonyl)anilino]-1,3,5-triazine restante sont consignés dans le tableau (II) suivant :

**Tableau (II)**

| Emulsion | UVINUL T 150 résiduel |
|---|---|
| Emulsion A (comparative) | 79 % |
| Emulsion B (comparative) | 63 % |
| Emulsion C (invention) | 98 % |
| Emulsion D (comparative) | 77 % |

## Revendications

1. Composition cosmétique et/ou dermatologique comprenant, dans un support cosmétiquement et/ou dermatologiquement acceptable, i) un dérivé de dibenzoylméthane, ii) au moins un dérivé de 1,3,5-triazine répondant à la formule (I) suivante : 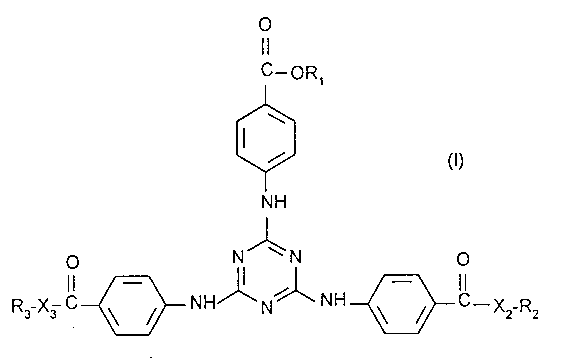 dans laquelle :
- X₂ et X₃, identiques ou différents, représentent l'oxygène ou le radical -NH- ;
- R₁, R₂ et R₃, identiques ou différents, sont choisis parmi : l'hydrogène ; un métal alcalin ; un radical ammonium éventuellement substitué par un ou plusieurs radicaux alkyles ou hydroxyalkyles ; un radical alkyle linéaire ou ramifié en C₁-C₁₈ ; un radical cycloalkyle en C₅-C₁₂ éventuellement substitué par un ou plusieurs radicaux alkyles en C₁-C₄ ; un radical polyoxyéthyléné comprenant de 1 à 6 unités d'oxyde d'éthylène et dont le groupe OH terminal est méthylé ; un radical de formule (II), (III) ou (IV) suivantes : 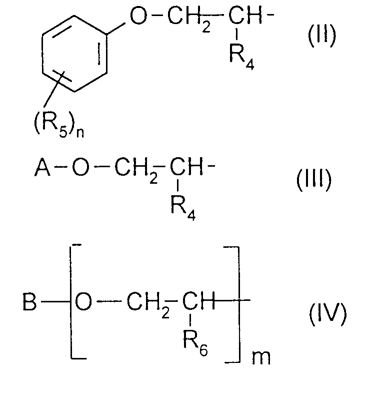 dans lesquelles :
- R₄ est l'hydrogène ou un radical méthyle ;
- R₅ est un radical alkyle en C₁-C₉ ;
- n est un nombre entier allant de 0 à 3 ;
- m est un nombre entier allant de 1 à 10 ;
- A est un radical alkyle en C₄-C₈ ou un radical cycloalkyle en C₅-C₈ ;
- B est choisi parmi : un radical alkyle linéaire ou ramifié en C₁-C₈ ; un radical cycloalkyle en C₅-C₈ ; un radical aryle éventuellement substitué par un ou plusieurs radicaux alkyles en C₁-C₄ ;
- R₆ est l'hydrogène ou un radical méthyle,
et iii) au moins un benzalmalonate de dialkyle de formule (V) suivante :
C₆(R₇)₃H₂-CH=C-[C(O)OR₈]₂ (V)
dans laquelle :
- R₇ représente indépendamment un atome d'hydrogène, un radical hydroxyle, un radical alkyle en C₁-C₈, un radical alcényle en C₂-C₈, un radical alcynyle en C₂-C₈, un radical alcoxy en C₁-C₈, un radical alcényloxy en C₂-C₈, un radical alcynyloxy en C₂-C₈,
- R₈ représente un radical alkyle en C₁-C₈.

2. Composition selon la revendication 1, caractérisée par le fait que le dérivé de 1,3,5-triazine est choisi parmi ceux de formule (I) présentant l'ensemble des caractéristiques suivantes :
- X₂ et X₃ sont identiques et représentent l'oxygène;
- R₁ est choisi parmi : un radical cycloalkyle en C₅-C₁₂ éventuellement substitué par un ou plusieurs radicaux alkyles en C₁-C₄ ; un radical de formule (II), (III) ou (IV) dans lesquelles :
- B est un radical alkyle en C₁-C₄ ;
- R₆ est le radical méthyle ;
- R₂ et R₃, identiques ou différents, sont choisis parmi : l'hydrogène ; un métal alcalin ; un radical ammonium éventuellement substitué par un ou plusieurs radicaux alkyles ou hydroxyalkyles ; un radical alkyle linéaire ou ramifié en C₁-C₁₈ ; un radical cycloalkyle en C₅-C₁₂ éventuellement substitué par un ou plusieurs radicaux alkyles en C₁-C₄ ; un radical de formule (II), (III) ou (IV) dans lesquelles :
- B est un radical alkyle en C₁-C₄ ;
- R₆ est le radical méthyle.

3. Composition selon la revendication 1, caractérisée par le fait que le dérivé de 1,3,5-triazine est choisi parmi ceux de formule (I) présentant l'ensemble des caractéristiques suivantes :
- X₂ et X₃ sont identiques et représentent le radical -NH- ;
- R₃ est choisi parmi : un radical alkyle linéaire ou ramifié en C₁-C₁₈ ; un radical cycloalkyle en C₅-C₁₂ éventuellement substitué avec un ou plusieurs radicaux alkyles en C₁-C₄ ;
- R₁ est choisi parmi : l'hydrogène ; un métal alcalin ; un radical ammonium ; un radical de formule (IV) ; un radical alkyle linéaire ou ramifié en C₁-C₁₈ ; un radical cycloalkyle en C₅-C₁₂ éventuellement substitué avec un ou plusieurs radicaux alkyles en C₁-C₄ ;
- R₂ est choisi parmi : un radical alkyle linéaire ou ramifié en C₁-C₁₈ ; un radical cycloalkyle en C₅-C₁₂ éventuellement substitué avec un ou plusieurs radicaux alkyles en C₁-C₄.

4. Composition selon la revendication 1, caractérisée par le fait que le dérivé de 1,3,5-triazine est choisi parmi ceux présentant l'ensemble des caractéristiques suivantes :
- X₂ est l'oxygène ;
- X₃ est le radical -NH- ;
- R₃ est choisi parmi : un radical alkyle linéaire ou ramifié en C₁-C₁₈ un radical cycloalkyle en C₅-C₁₂ éventuellement substitué avec un ou plusieurs radicaux alkyles en C₁-C₄ ;
- R₁ est choisi parmi : l'hydrogène ; un métal alcalin ; un radical ammonium ; un radical de formule (IV) ; un radical alkyle linéaire ou ramifié en C₁-C₁₈ ; un radical cycloalkyle en C₅-C₁₂ éventuellement substitué avec un ou plusieurs radicaux alkyles en C₁-C₄ ;
- R₂ est choisi parmi l'hydrogène ; un métal alcalin ; un radical ammonium ; un radical de formule (IV) ; un radical alkyle linéaire ou ramifié en C₁-C₁₈ ; un radical cycloalkyle en C₅-C₁₂ éventuellement substitué avec un ou plusieurs radicaux alkyles en C₁-C₄.

5. Composition selon la revendication 4, caractérisée par le fait que le dérivé de 1,3,5-triazine est celui répondant à la formule suivante : 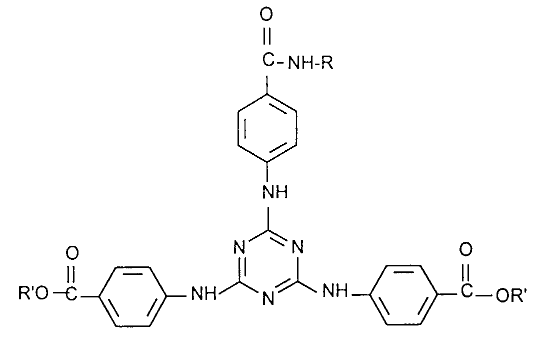 dans laquelle R 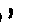 désigne un radical éthyl-2 hexyle et R désigne un radical ter. butyle.

6. Composition selon la revendication 1, caractérisée par le fait que le dérivé de 1,3,5-triazine est choisi parmi ceux présentant l'ensemble des caractéristiques suivantes :
- X₂ et X₃ sont identiques et représentent l'oxygène ;
- R₁, R₂ et R₃ sont identiques et représentent un radical alkyle en C₆-C₁₂ ou un radical polyoxyéthyléné comprenant de 1 à 6 unités d'oxyde d'éthylène et dont le groupe OH terminal est méthylé.

7. Composition selon la revendication 6, caractérisée par le fait que le dérivé de 1,3,5-triazine est celui répondant à la formule suivante : 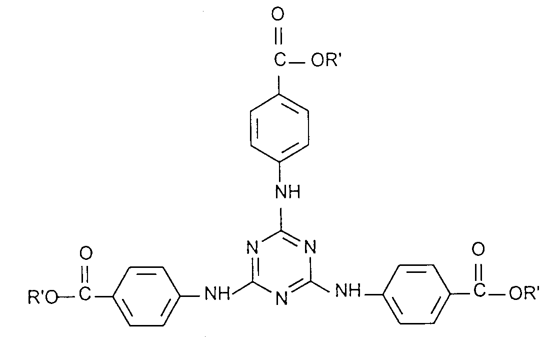 dans laquelle R 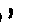 désigne un radical 2-éthyl hexyle.

8. Composition selon l'une quelconque des revendications 1 à 7, caractérisée par le fait que le dérivé de 1,3,5-triazine est présent dans la composition à une teneur allant de 0,5 % à 20 % en poids, par rapport au poids total de la composition, de préférence de 1 % à 10 % en poids, par rapport au poids total de la composition.

9. Composition selon l'une quelconque des revendications 1 à 8, caractérisée par le fait que ledit benzalmalonate de dialkyle est choisi parmi ceux répondant à la formule (VI) ci-dessous: 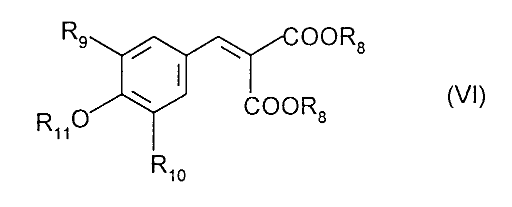 dans laquelle :
- R₈ a la même signification qu'à la formule (V) ci-dessus,
- R₉ et R₁₀, identiques ou différents, représentent indépendamment un atome d'hydrogène ou un radical alcoxy en C₁-C₈,
- R₁₁ représente un radical alkyle en C₁-C₈.

10. Composition selon l'une quelconque des revendications précédentes, caractérisée par le fait le benzalmalonate de dialkyle est choisi parmi les composés suivants :
- 4 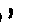 -méthoxy-benzalmalonate de diéthyle,
- 4 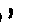 -tert.-butoxy-benzalmalonate de diéthyle,
- 4 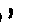 -méthoxy-benzalmalonate de diisopropyle,
- 4 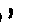 -méthoxy-benzalmalonate de di-2-éthylhexyle,
- 4 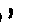 -n-butoxy-3'-méthoxy-benzalmalonate de diéthyle,
- 4 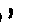 -n-butoxy-3'-méthoxy-benzalmalonate de diisopropyle,
- 3 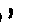 ,4 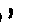 -diméthoxy-benzalmalonate de di-2-éthylhexyle,
- 3 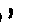 ,4 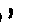 ,5 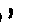 -triméthoxy-benzalmalonate de diisopropyle,
- 4 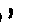 -méthoxy-benzalmalonate de diisoamyle,
- 4 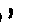 -n-hexyloxy-benzalmalonate de diéthyle.

11. Composition selon l'une quelconque des revendications précédentes, caractérisée par le fait que le benzalmalonate de dialkyle est présent dans la composition à une teneur au moins égale à 0,5 % en poids, par rapport au poids total de la composition, de préférence de 0,5 % à 20 % en poids, par rapport au poids total de la composition.

12. Composition selon l'une quelconque des revendications précédentes, caractérisée par le fait que le dérivé de dibenzoylméthane est choisi parmi :
- le 2-méthyldibenzoylméthane,
- le 4-méthyldibenzoylméthane,
- le 4-isopropyldibenzoylméthane,
- le 4-tert.-butyldibenzoylméthane,
- le 2,4-diméthyldibenzoylméthane,
- le 2,5-diméthyldibenzoylméthane,
- le 4,4'-diisopropyldibenzoylméthane,
- le 4-tert.-butyl-4'-méthoxydibenzoylméthane,
- le 2-méthyl-5-isopropyl-4'-méthoxydibenzoylméthane,
- le 2-méthyl-5-tert.-butyl-4'-méthoxydibenzoylméthane,
- le 2,4-diméthyl-4'-méthoxydibenzoylméthane,
- le 2,6-diméthyl-4-tert.-butyl-4'-méthoxydibenzoylméthane,
- le 4,4 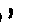 -diméthoxydibenzoylméthane.

13. Composition selon l'une quelconque des revendications précédentes, caractérisée par le fait que le dérivé de dibenzoylméthane est le 4-tert-butyl-4 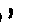 -méthoxydibenzoylméthane.

14. Composition selon l'une quelconque des revendications précédentes, caractérisée par le fait que le dérivé de dibenzoylméthane est présent dans la composition à une teneur allant de 0,2 % à 15 % en poids, par rapport au poids total de la composition, de préférence de 0,2 % à 10 % en poids, par rapport au poids total de la composition.

15. Composition selon l'une quelconque des revendications précédentes, caractérisée par le fait qu'elle est sous la forme d'une émulsion huile-dans-eau.

16. Utilisation d'un benzalmalonate de dialkyle tel que défini à l'une quelconque des revendications 1, 9 ou 10 dans, ou pour la fabrication de, compositions cosmétiques et/ou pour la fabrication de compositions dermatologiques contenant un dérivé de dibenzoylméthane tel que défini à l'une quelconque des revendications 1, 12 ou 13 en association avec au moins un dérivé de 1,3,5-triazine tel que défini à l'une quelconque des revendications 1 à 7 en vue d'améliorer dans lesdites compositions la stabilité au rayonnement UV dudit dérivé de 1,3,5-triazine.

17. Procédé pour améliorer la stabilité au rayonnement UV des compositions cosmétiques et/ou dermatologiques comprenant un dérivé de dibenzoylméthane tel que défini à l'une quelconque des revendications 1, 12 ou 13 et un dérivé de 1,3,5-triazine tel que défini à l'une quelconque des revendications 1 à 7, caractérisé par le fait qu'il consiste à introduire dans lesdites compositions une quantité efficace d'un benzalmalonate de dialkyle tel que défini à l'une quelconque des revendications 1, 9 ou 10.

## Claims

1. Cosmetic and/or dermatological composition comprising, in a cosmetically and/or dermatologically acceptable support, i) a dibenzoylmethane derivative, ii) at least one 1,3,5-triazine derivative corresponding to formula (I) below: 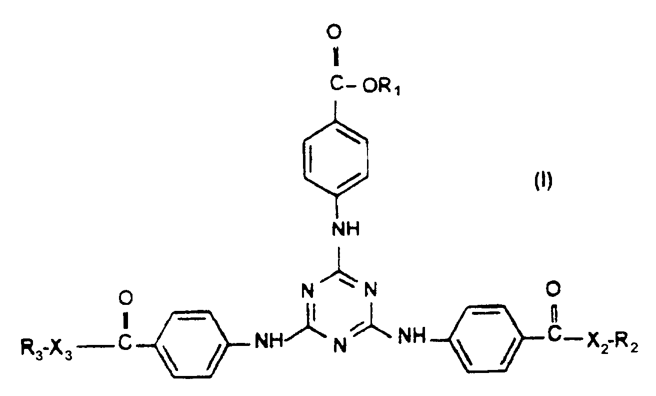 in which:
- X₂ and X₃, which may be identical or different, represent oxygen or an -NH- radical;
- R₁, R₂ and R₃, which may be identical or different, are chosen from: hydrogen; an alkali metal; an ammonium radical optionally substituted with one or more alkyl or hydroxyalkyl radicals; a linear or branched C₁-C₁₈ alkyl radical; a C₅-C₁₂ cycloalkyl radical optionally substituted with one or more C₁-C₄ alkyl radicals; a polyoxyethylenated radical comprising from 1 to 6 ethylene oxide units and whose terminal OH group is methylated; a radical of formula (II), (III) or (IV) below: 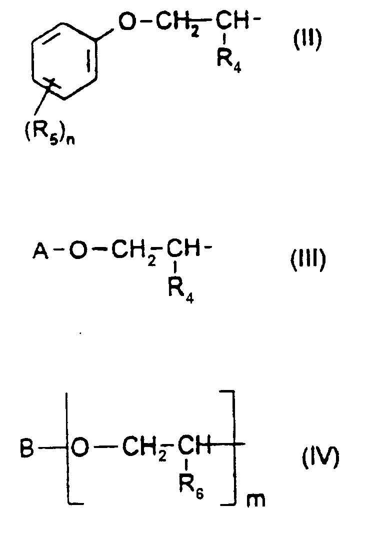 in which:
- R₄ is hydrogen or a methyl radical;
- R₅ is a C₁-C₉ alkyl radical;
- n is an integer ranging from 0 to 3;
- m is an integer ranging from 1 to 10;
- A is a C₄-C₈ alkyl radical or a C₅-C₈ cycloalkyl radical;
- B is chosen from: a linear or branched C₁-C₈ alkyl radical; a C₅-C₈ cycloalkyl radical; an aryl radical optionally substituted with one or more C₁-C₄ alkyl radicals;
- R₆ is hydrogen or a methyl radical,
and iii) at least one dialkyl benzalmalonate of formula (V) below:
C₆(R₇)₃H₂-CH=C-[C(O)OR₈]₂ (V)
in which:
- R₇ represents, independently, a hydrogen atom, a hydroxyl radical, a C₁-C₈ alkyl radical, a C₂-C₈ alkenyl radical, a C₂-C₈ alkynyl radical, a C₁-C₈ alkoxy radical, a C₂-C₈ alkenyloxy radical or a C₂-C₈ alkynyloxy radical,
- R₈ represents a C₁-C₈ alkyl radical.

2. Composition according to Claim 1, characterized in that the 1,3,5-triazine derivative is chosen from those of formula (I) having all of the following characteristics:
- X₂ and X₃ are identical and represent oxygen;
- R₁ is chosen from: a C₅-C₁₂ cycloalkyl radical optionally substituted with one or more C₁-C₄ alkyl radicals; a radical of formula (II), (III) or (IV) in which:
- B is a C₁-C₄ alkyl radical;
- R₆ is a methyl radical;
- R₂ and R₃, which may be identical or different, are chosen from: hydrogen; an alkali metal; an ammonium radical optionally substituted with one or more alkyl or hydroxyalkyl radicals; a linear or branched C₁-C₁₈ alkyl radical; a C₅-C₁₂ cycloalkyl radical optionally substituted with one or more C₁-C₄ alkyl radicals; a radical of formula (II), (III) or (IV) in which:
- B is a C₁-C₄ alkyl radical;
- R₆ is a methyl radical.

3. Composition according to Claim 1, characterized in that the 1,3,5-triazine derivative is chosen from those of formula (I) having all of the following characteristics:
- X₂ and X₃ are identical and represent the -NH- radical;
- R₃ is chosen from: a linear or branched C₁-C₁₈ alkyl radical; a C₅-C₁₂ cycloalkyl radical optionally substituted with one or more C₁-C₄ alkyl radicals;
- R₁ is chosen from: hydrogen; an alkali metal; an ammonium radical; a radical of formula (IV); a linear or branched C₁-C₁₈ alkyl radical; a C₅-C₁₂ cycloalkyl radical optionally substituted with one or more C₁-C₄ alkyl radicals;
- R₂ is chosen from: a linear or branched C₁-C₁₈ alkyl radical; a C₅-C₁₂ cycloalkyl radical optionally substituted with one or more C₁-C₄ alkyl radicals.

4. Composition according to Claim 1, characterized in that the 1,3,5-triazine derivative is chosen from those having all of the following characteristics:
- X₂ is oxygen;
- X₃ is the -NH- radical;
- R₃ is chosen from: a linear or branched C₁-C₁₈ alkyl radical; a C₅-C₁₂ cycloalkyl radical optionally substituted with one or more C₁-C₄ alkyl radicals;
- R₁ is chosen from: hydrogen; an alkali metal; an ammonium radical; a radical of formula (IV); a linear or branched C₁-C₁₈ alkyl radical; a C₅-C₁₂ cycloalkyl radical optionally substituted with one or more C₁-C₄ alkyl radicals;
- R₂ is chosen from: hydrogen; an alkali metal; an ammonium radical; a radical of formula (IV); a linear or branched C₁-C₁₈ alkyl radical; a C₅-C₁₂ cycloalkyl radical optionally substituted with one or more C₁-C₄ alkyl radicals.

5. Composition according to Claim 4, characterized in that the 1,3,5-triazine derivative is that corresponding to the following formula: 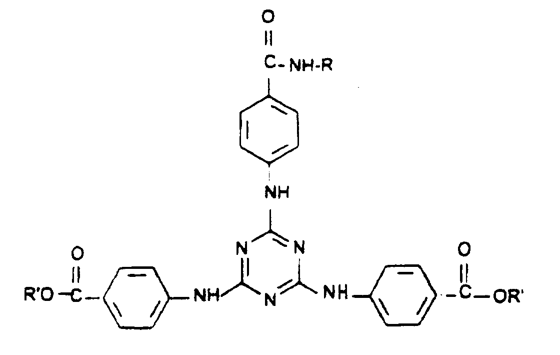 in which R' denotes a 2-ethylhexyl radical and R denotes a tert-butyl radical.

6. Composition according to Claim 1, characterized in that the 1,3,5-triazine derivative is chosen from those having all of the following characteristics:
- X₂ and X₃ are identical and represent oxygen;
- R₁, R₂ and R₃ are identical and represent a C₆-C₁₂ alkyl radical or a polyoxyethylenated radical comprising from 1 to 6 ethylene oxide units and in which the terminal OH group is methylated.

7. Composition according to Claim 6, characterized in that the 1,3,5-triazine derivative is that corresponding to the following formula: 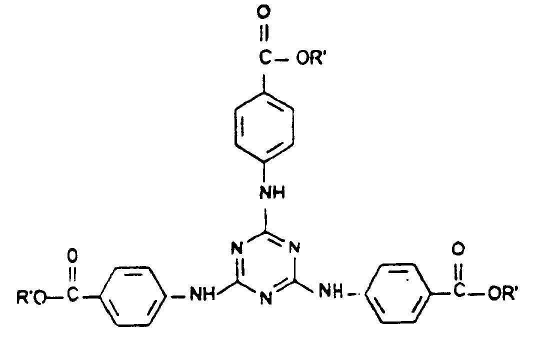 in which R' denotes a 2-ethylhexyl radical.

8. Composition according to any one of Claims 1 to 7, characterized in that the 1,3,5-triazine derivative is present in the composition in a proportion ranging from 0.5% to 20% by weight, relative to the total weight of the composition, preferably from 1% to 10% by weight, relative to the total weight of the composition.

9. Composition according to any one of Claims 1 to 8, characterized in that the said dialkyl benzalmalonate is chosen from those corresponding to formula (VI) below: 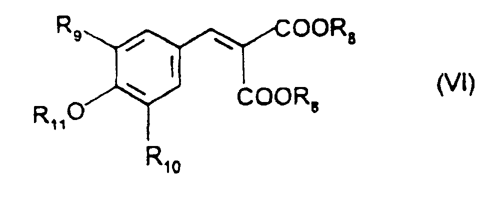 in which:
- R₈ has the same meaning as in formula (V) above,
- R₉ and R₁₀, which may be identical or different, independently represent a hydrogen atom or a C₁-C₈ alkoxy radical,
- R₁₁ represents a C₁-C₈ alkyl radical.

10. Composition according to any one of the preceding claims, characterized in that the dialkyl benzalmalonate is chosen from the following compounds:
- diethyl 4'-methoxybenzalmalonate,
- diethyl 4'-tert-butoxybenzalmalonate,
- diisopropyl 4'-methoxybenzalmalonate,
- bis(2-ethylhexyl) 4'-methoxybenzalmalonate,
- diethyl 4'-n-butoxy-3'-methoxybenzalmalonate,
- diisopropyl 4'-n-butoxy-3'-methoxybenzalmalonate,
- bis(2-ethylhexyl) 3',4'-dimethoxybenzalmalonate,
- diisopropyl 3',4',5'-trimethoxybenzalmalonate,
- diisoamyl 4'-methoxybenzalmalonate,
- diethyl 4'-n-hexyloxybenzalmalonate.

11. Composition according to any one of the preceding claims, characterized in that the dialkyl benzalmalonate is present in the composition in a proportion at least equal to 0.5% by weight, relative to the total weight of the composition, preferably from 0.5% to 20% by weight, relative to the total weight of the composition.

12. Composition according to any one of the preceding claims, characterized in that the dibenzoylmethane derivative is chosen from:
- 2-methyldibenzoylmethane,
- 4-methyldibenzoylmethane,
- 4-isopropyldibenzoylmethane,
- 4-tert-butyldibenzoylmethane,
- 2,4-dimethyldibenzoylmethane,
- 2,5-dimethyldibenzoylmethane,
- 4,4'-diisopropyldibenzoylmethane,
- 4-tert-butyl-4'-methoxydibenzoylmethane,
- 2-methyl-5-isopropyl-4'-methoxydibenzoylmethane,
- 2-methyl-5-tert-butyl-4'-methoxydibenzoylmethane,
- 2,4-dimethyl-4'-methoxydibenzoylmethane,
- 2,6-dimethyl-4-tert-butyl-4'-methoxydibenzoylmethane,
- 4,4'-dimethoxydibenzoylmethane.

13. Composition according to any one of the preceding claims, characterized in that the dibenzoylmethane derivative is 4-tert-butyl-4'-methoxydibenzoylmethane.

14. Composition according to any one of the preceding claims, characterized in that the dibenzoylmethane derivative is present in the composition in a proportion ranging from 0.2% to 15% by weight, relative to the total weight of the composition, preferably from 0.2% to 10% by weight, relative to the total weight of the composition.

15. Composition according to any one of the preceding claims, characterized in that it is in the form of an oil-in-water emulsion.

16. Use of a dialkyl benzalmalonate as defined in any one of Claims 1, 9 or 10 in, or for the manufacture of, cosmetic compositions and/or for the manufacture of dermatological compositions containing a dibenzoylmethane derivative as defined in any one of Claims 1, 12 or 13, in combination with at least one 1,3,5-triazine derivative as defined in any one of Claims 1 to 7, in order to enhance the stability to UV radiation of the said 1,3,5-triazine derivative in the said compositions.

17. Process for enhancing the stability to UV radiation of the cosmetic and/or dermatological compositions comprising a dibenzoylmethane derivative as defined in any one of Claims 1, 12 or 13, and a 1,3,5-triazine derivative as defined in any one of Claims 1 to 7, characterized in that it consists in introducing into the said compositions an effective amount of a dialkyl benzalmalonate as defined in any one of Claims 1, 9 or 10.

## Patentansprüche

1. Kosmetische und/oder dermatologische Zusammensetzung, die in einem kosmetisch und/oder dermatologisch akzeptablen Träger enthält:
i) ein Dibenzoylmethanderivat,
ii) mindestens ein 1,3,5-Triazinderivat der folgenden Formel (I): 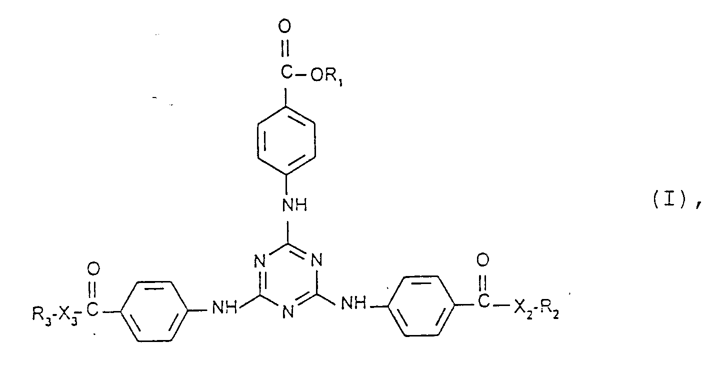 worin:
- die Gruppen X₂ und X₃, die identisch oder voneinander verschieden sind, Sauerstoff oder die Gruppe -NH- bedeuten; und
- die Gruppen R₁, R₂ und R₃, die identisch oder voneinander verschieden sind, ausgewählt sind unter: Wasserstoff; einem Alkalimetall; einer Ammoniumgruppe, die gegebenenfalls mit einer oder mehreren Alkyl- oder Hydroxyalkylgruppen substituiert ist; einer geradkettigen oder verzweigten C₁₋₁₈-Alkylgruppe; einer C₅₋₁₂-Cycloalkylgruppe, die gegebenenfalls mit einer oder mehreren C₁₋₄-Alkylgruppen substituiert ist; einer Polyoxyethylengruppe, die 1 bis 6 Ethylenoxideinheiten aufweist und deren OH-Endgruppe methyliert ist; einer Gruppe der folgenden Formeln (II), (III) oder (IV): 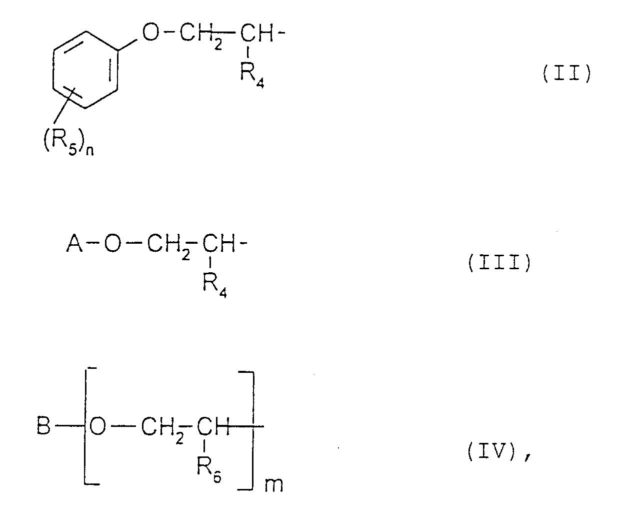 worin bedeuten:
- R₄ Wasserstoff oder Methyl;
- R₅ eine C₁₋₉-Alkylgruppe;
- n Null oder eine ganze Zahl von 1 bis 3;
- m eine ganze Zahl im Bereich von 1 bis 10;
- A eine C₄₋₈-Alkylgruppe oder eine C₅₋₈-Cycloalkylgruppe;
- B eine geradkettige oder verzweigte C₁₋₈-Alkylgruppe; eine C₅₋₈-Cycloalkylgruppe; eine Arylgruppe, die gegebenenfalls mit einer oder mehreren C₁₋₄-Alkylgruppe substituiert ist;
- R₆ Wasserstoff oder Methyl;
und iii) mindestens ein Dialkylbenzalmalonat der folgenden Formel (V):
C₆(R₇)₃H₂-CH=C-[C(O)OR₈]₂ (V)
worin bedeuten:
- die Gruppen R₇ unabhängig voneinander Wasserstoff, Hydroxy, C₁₋₈-Alkyl, C₂₋₈-Alkenyl, C₂₋₈-Alkinyl, C₁₋₈-Alkoxy, C₂₋₈-Alkenyloxy, C₂₋₈-Alkinyloxy;
- R₈ C₁₋₈-Alkyl.

2. Zusammensetzung nach Anspruch 1, dadurch gekennzeichnet, daß das 1,3,5-Triazinderivat unter den Derivaten der Formel (I) ausgewählt ist, die die gesamten folgenden Eigenschaften aufweisen:
- X₂ und X₃ sind identisch und bedeuten Sauerstoff;
- R₁ ist ausgewählt unter: einer C₅₋₁₂-Cycloalkylgruppe, die gegebenenfalls mit einer oder mehreren C₁₋₄-Alkylgruppen substituiert ist; einer Gruppe der Formel (II), (III) oder (IV), worin bedeuten:
- B eine C₁₋₄-Alkylgruppe;
- R₆ Methyl;
- R₂ und R₃, die identisch oder voneinander verschieden sind, sind ausgewählt unter: Wasserstoff; einem Alkalimetall; einer Ammoniumgruppe, die gegebenenfalls mit einer oder mehreren Alkyl- oder Hydroxyalkylgruppen substituiert ist; einer geradkettigen oder verzweigten C₁₋₁₈-Alkylgruppe; einer C₅-₁₂-Cycloalkylgruppe, die gegebenenfalls mit einer oder mehreren C₁₋₄-Alkylgruppen substituiert ist; einer Gruppe der Formel (II), (III) oder (IV), worin bedeuten:
- B eine C₁₋₄-Alkylgruppe;
- R₆ Methyl.

3. Zusammensetzung nach Anspruch 1, dadurch gekennzeichnet, daß das 1,3,5,-Triazinderivat unter den Derivaten der Formel (I) ausgewählt ist, die die gesamten folgenden Eigenschaften aufweisen:
- X₂ und X₃ sind identisch und bedeuten die Gruppe -NH-;
- R₃ ist ausgewählt unter einer geradkettigen oder verzweigten C₁₋₁₈-Alkylgruppe; einer C₅₋₁₂-Cycloalkylgruppe, die gegebenenfalls mit einer oder mehreren C₁₋₄-Alkylgruppen substituiert ist;
- R₁ ist ausgewählt unter Wasserstoff; einem Alkalimetall; einer Ammoniumgruppe; einer Gruppe der Formel (IV); einer geradkettigen oder verzweigten C₁₋₁₈-Alkylgruppe; einer C₅-₁₂-Cycloalkylgruppe, die gegebenenfalls mit einer oder mehreren C₁₋₄-Alkylgruppen substituiert ist;
- R₂ ist ausgewählt unter: einer geradkettigen oder verzweigten C₁₋₁₈-Alkylgruppe; einer C₅₋₁₂-Cycloalkylgruppe, die gegebenenfalls mit einer oder mehreren C₁₋₄-Alkylgruppen substituiert ist.

4. Zusammensetzung nach Anspruch 1, dadurch gekennzeichnet, daß das 1,3,5-Triazinderivat unter den Derivaten ausgewählt ist, die die gesamten folgenden Eigenschaften aufweisen:
- X₂ ist Sauerstoff;
- X₃ ist die Gruppe -NH-;
- R₃ ist ausgewählt unter einer geradkettigen oder verzweigten C₁₋₁₈-Alkylgruppe; einer C₅₋₁₂-Cycloalkylgruppe, die gegebenenfalls mit einer oder mehreren C₁₋₄-Alkylgruppen substituiert ist;
- R₁ ist ausgewählt unter Wasserstoff; einem Alkalimetall; einer Ammoniumgruppe; einer Gruppe der Formel (IV); einer geradkettigen oder verzweigten C₁₋₁₈-Alkylgruppe; einer C₅₋₁₂-Cycloalkylgruppe, die gegebenenfalls mit einer oder mehreren C₁₋₄-Alkylgruppen substituiert ist;
- R₂ ist ausgewählt unter Wasserstoff; einem Alkalimetall; einer Ammoniumgruppe; einer Gruppe der Formel (IV); einer geradkettigen oder verzweigten C₁₋₁₈-Alkylgruppe; einer C₅₋₁₂-Cycloalkylgruppe, die gegebenenfalls mit einer oder mehreren C₁₋₄-Alkylgruppen substituiert ist.

5. Zusammensetzung nach Anspruch 4, dadurch gekennzeichnet, daß das 1,3,5-Triazinderivat der folgenden Formel entspricht: 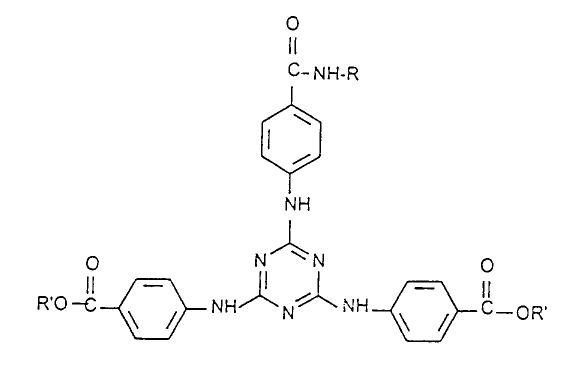 worin die Gruppen R' die 2-Ethylhexylgruppe und R die tert.-Butylgruppe bedeuten.

6. Zusammensetzung nach Anspruch 1, dadurch gekennzeichnet, daß das 1,3,5-Triazinderivat unter den Derivaten ausgewählt ist, die alle folgenden Eigenschaften aufweisen:
- X₂ und X₃ sind identisch und bedeuten Sauerstoff;
- R₁, R₂ und R₃ sind identisch und bedeuten eine C₆₋₁₂-Alkylgruppe oder eine Polyoxyethylengruppe mit 1 bis 6 Ethylenoxideinheiten, deren OH-Endgruppe methyliert ist.

7. Zusammensetzung nach Anspruch 6, dadurch gekennzeichnet, daß das 1,3,5-Triazinderivat der folgenden Formel entspricht: 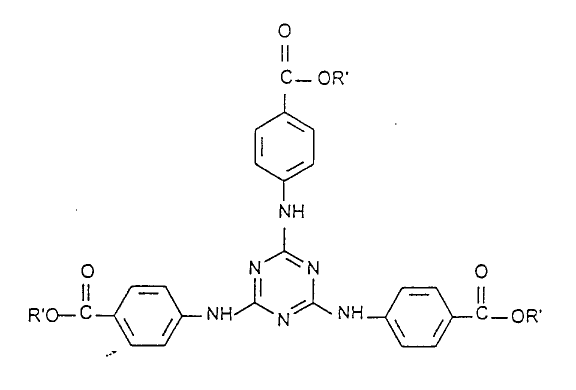 worin die Gruppen R' die 2-Ethylhexylgruppe bedeuten.

8. Zusammensetzung nach einem der Ansprüche 1 bis 7, dadurch gekennzeichnet, daß das 1,3,5-Triazinderivat in der Zusammensetzung in einem Mengenanteil von 0,5 bis 20 Gew.-%, bezogen auf das Gesamtgewicht der Zusammensetzung, und vorzugsweise im Bereich von 1 bis 10 Gew.-%, bezogen auf das Gesamtgewicht der Zusammensetzung, vorliegt.

9. Zusammensetzung nach einem der Ansprüche 1 bis 8, dadurch gekennzeichnet, daß das Dialkylbenzalmalonat unter den Verbindungen der folgenden Formel (VI) ausgewählt ist: 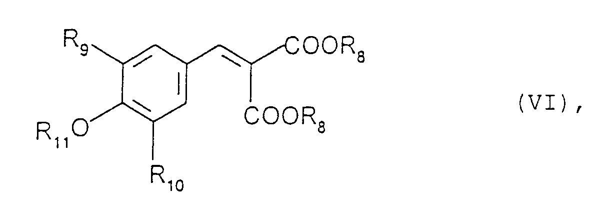 worin:
- R₈ die in der obengenannten Formel (V) angegebene Bedeutung aufweist,
- R₉ und R₁₀, die identisch oder voneinander verschieden sind, unabhängig voneinander Wasserstoff oder eine C₁₋₈-Alkoxygruppe bedeuten, und
- R₁₁ eine C₁₋₈-Alkylgruppe bedeutet.

10. Zusammensetzung nach einem der vorhergehenden Ansprüche, dadurch gekennzeichnet, daß das Dialkylbenzalmalonat unter den folgenden Verbindungen ausgewählt ist:
- Diethyl-4'-methoxy-benzalmalonat,
- Diethyl-4'-*tert*.-butoxy-benzalmalonat,
- Diisopropyl-4'-methoxy-benzalmalonat,
- Di-2-ethylhexyl-4'-methoxy-benzalmalonat,
- Diethyl-4'-n-butoxy-3'-methoxy-benzalmalonat,
- Diisopropyl-4'-n-butoxy-3'-methoxy-benzalmalonat,
- Di-2-ethylhexyl-3',4'-dimethoxy-benzalmalonat,
- Diisopropyl-3',4',5'-trimethoxy-benzalmalonat,
- Diisoamyl-4'-methoxy-benzalmalonat und
- Diethyl-4'-n-hexyloxy-benzalmalonat.

11. Zusammensetzung nach einem der vorhergehenden Ansprüche, dadurch gekennzeichnet, daß das Dialkylbenzalmalonat in der Zusammensetzung in einem Mengenanteil von mindestens 0,5 Gew.-%, bezogen auf das Gesamtgewicht der Zusammensetzung, und vorzugsweise im Bereich von 0,5 bis 20 Gew.-%, bezogen auf das Gesamtgewicht der Zusammensetzung, vorliegt.

12. Zusammensetzung nach einem der vorhergehenden Ansprüche, dadurch gekennzeichnet, daß das Dibenzoylmethanderivat ausgewählt ist unter:
- 2-Methyl-dibenzoylmethan,
- 4-Methyl-dibenzoylmethen,
- 4-Isoproyl-dibenzoylmethan,
- 4-*tert*.-Butyl-dibenzoylmethan,
- 2,4-Dimethyl-dibenzoylmethan,
- 2,5-Dimethyl-dibenzoylmethan,
- 4,4'-Diisopropyl-dibenzoylmethan,
- 4-*tert*.-Butyl-4'-methoxy-dibenzoylmethan,
- 2-Methyl-5-isopropyl-4'-methoxy-dibenzoylmethan,
- 2-Methyl-5-*tert*.-butyl-4'-methoxy-dibenzoylmethan,
- 2,4-Dimethyl-4'-methoxy-dibenzoylmethan,
- 2,6-Dimethyl-4-*tert*.-butyl-4'-methoxydibenzoylmethan, und
- 4,4'-Dimethoxy-dibenzoylmethan.

13. Zusammensetzung nach einem der vorhergehenden Ansprüche, dadurch gekennzeichnet, daß das Dibenzoylmethanderivat das 4-*tert*.-Butyl-4'-methoxydibenzoylmethan ist.

14. Zusammensetzung nach einem der vorhergehenden Ansprüche, dadurch gekennzeichnet, daß das Dibenzoylmethanderivat in der Zusammensetzung in einem Mengenanteil von 0,2 bis 15 Gew.-%, bezogen auf das Gesamtgewicht der Zusammensetzung, und vorzugsweise im Bereich von 0,2 bis 10 Gew.-%, bezogen auf das Gesamtgewicht der Zusammensetzung, vorliegt.

15. Zusammensetzung nach einem der vorhergehenden Ansprüche, dadurch gekennzeichnet, daß sie in Form einer Öl-in-Wasser-Emulsion vorliegt.

16. Verwendung eines Dialkylbenzalmalonats nach einem der Ansprüche 1, 9 oder 10 in kosmetischen Zusammensetzungen oder zur Herstellung von kosmetischen Zusammensetzungen und/oder zur Herstellung von dermatologischen Zusammensetzungen, die ein Dibenzoylmethanderivat nach einem der Ansprüche 1, 12 oder 13 in Kombination mit mindestens einem 1,3,5-Triazinderivat nach einem der Ansprüche 1 bis 7 enthalten, um die Stabilität des 1,3,5-Triazinderivats gegenüber UV-Strahlung in diesen Zusammensetzungen zu verbessern.

17. Verfahren zur Verbesserung der Stabilität von kosmetischen und/oder dermatologischen Zusammensetzungen gegen UV-Strahlung, die ein Dibenzoylmethanderivat nach einem der Ansprüche 1, 12 oder 13 und ein 1,3,5-Triazinderivat nach einem der Ansprüche 1 bis 7 enthalten, dadurch gekennzeichnet, daß es darin besteht, eine wirksame Menge eines Dialkylbenzalmalonats nach einem der Ansprüche 1, 9 oder 10 in die Zusammensetzungen einzuarbeiten.
